(19)

Europäisches
Patentamt
European
Patent Office
Office européen
des brevets

(11) **EP 2 591 727 A1**

(12) **EUROPEAN PATENT APPLICATION**
published in accordance with Art. 153(4) EPC

(43) Date of publication:
**15.05.2013 Bulletin 2013/20**

(21) Application number: **11803513.8**

(22) Date of filing: **01.07.2011**

(51) Int Cl.:
**A61B 6/00** (2006.01)

(86) International application number:
**PCT/JP2011/065153**

(87) International publication number:
**WO 2012/005179 (12.01.2012 Gazette 2012/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.07.2010 JP 2010156574
02.02.2011 JP 2011020502**

(71) Applicant: **FUJIFILM Corporation
Tokyo 106-8620 (JP)**

(72) Inventor: **ISHII, Hiroyasu
Ashigarakami-gun
Kanagawa 258-8538 (JP)**

(74) Representative: **Klunker . Schmitt-Nilson . Hirsch
Destouchesstraße 68
80796 München (DE)**

(54) **RADIOGRAPHIC IMAGING SYSTEM AND IMAGE PROCESSING METHOD OF SAME**

(57) An X-ray imaging system for preventing deterioration in image quality of a phase contrast image due to streak noise occurred in a direction corresponding to a signal line of an X-ray image detector. The X-ray imaging system is composed of an X-ray source, first and second absorption-type gratings, and a flat panel detector (FPD). Image captures are performed with the second absorption-type grating moved in X direction orthogonal to its grating line, relative to the first absorption-type grating. The FPD has pixels arranged in the X direction and Y direction orthogonal to the X direction. A group of the pixels arranged in the X direction are connected to a single signal line that reads out charge. A differential phase image producing section produces a differential phase image based on pieces of image data obtained from the respective image captures. A phase contrast image producing section integrates the differential phase image, produced by the differential phase image producing section, in a direction corresponding to the signal line of the FPD to produce a phase contrast image.

FIG. 1

EP 2 591 727 A1

**Description**

TECHNICAL FIELD

The present invention relates to a radiation imaging system using radiation to capture an image of a subject and an image processing method for use in a radiation imaging system, and particularly to a radiation imaging system using a fringe scanning method and an image processing method for use in a radiation imaging system using a fringe scanning method.

BACKGROUND ART

Radiation, for example, X-rays, is attenuated depending on an atomic number of an element constituting a substance, and density and thickness of a substance. By taking advantage of such properties, the X-rays are used as a probe for examining the inside of a subject in medical diagnoses and non-destructive inspections.

In a common X-ray imaging system, a subject is disposed between an X-ray source for emitting the X-rays and an X-ray image detector for detecting the X-rays, and a transmission image of the subject is captured. The X-rays, emitted from the X-ray source toward the X-ray image detector, are attenuated (absorbed) by a substance, disposed on a path toward the X-ray image detector, by an amount corresponding to differences in properties (the atomic number, the density, and the thickness) of the substance. Then the X-rays are incident on each pixel (X-ray conversion element) of the X-ray image detector. As a result, the X-ray image detector detects and images an X-ray absorption image of the subject. Stimulable phosphor panels and flat panel detectors (FPDs) using semiconductor circuits are widely used as the X-ray image detectors.

The X-ray absorption performance of the substance decreases as the atomic number of the element constituting the substance decreases. This causes a problem that a sufficient contrast cannot be obtained in the X-ray absorption image of living soft tissue or soft materials. For example, a cartilage portion constituting a joint of a human body and synovial fluid surrounding the cartilage portion are mainly composed of water, so that there is little difference between their amounts of X-ray absorption, resulting in little difference in contrast.

Recently, X-ray phase imaging has been studied actively to solve the above problem. The X-ray phase imaging is used to obtain an image (hereafter referred to as the phase contrast image) based on phase shifts (angular changes), instead of intensity changes, of the X-rays caused by the subject. Generally, when the X-rays are incident on the subject, the subject interacts with the phase of the X-rays more strongly than with the intensity of the X-rays. Thus, the X-ray phase imaging using phase difference provides a high contrast image even if the subject has low X-ray absorption properties. An X-ray imaging system using an X-ray Talbot interferometer is known as one type of the X-ray phase imaging. The X-ray Talbot interferometer is composed of two transmission diffraction gratings and an X-ray image detector (see, for example, patent document 1 and non-patent document 1)

In the X-ray Talbot interferometer, the first diffraction grating is disposed behind the subject. The second diffraction grating is disposed downstream of the first diffraction grating by a Talbot length. The Talbot length is determined by a grating pitch of the first diffraction grating and an X-ray wavelength. The X-ray image detector is disposed behind the second diffraction grating. The Talbot length is a distance at which the X-rays passed through the first diffraction grating form a self-image (fringe image) due to Talbot effect. The self image is modulated by the phase shift of the X-rays caused by the subject disposed between the X-ray source and the first diffraction grating.

In the X-ray imaging system, the intensity of the fringe image is modulated by the superposition of the self image of the first diffraction grating onto the second diffraction grating. The phase contrast image of the subject is obtained from the changes in the fringe image caused by the subject with the use of a fringe scanning method. In the fringe scanning method, the second diffraction grating is translationally moved (scanned) at a scanning pitch, being a fraction of the grating pitch, relative to the first diffraction grating in a direction substantially in parallel with the first grating and substantially vertical to a direction of a grating line of the first diffraction grating. The image is captured every time the second diffraction grating is moved to a subsequent scanning position. A differential phase image is obtained from a phase shift value of the intensity change, relative to the scanning position, of pixel data of each pixel with the use of the X-ray image detector. The differential phase image corresponds to angular distribution of the X-rays refracted by the subject. The phase contrast image is produced by integrating the differential phase image in the direction of the fringe scanning. Note that the intensity of the image data is modulated periodically by the scanning. A set of the image data relative to the above-described scanning is referred to as an "intensity modulation signal". The fringe scanning method is also employed in imaging apparatuses using laser (see, for example, non-patent document 2).

PRIOR ART DOCUMENTS

Patent Document

Patent Document 1: Patent No. 4445397

Non-Patent Documents

Non-Patent Document 1: C. David et al., Applied Physics Letters, Vol.81, No.17, October 2002, page 3287
Non-Patent Document 2: Hector Canabal et al., Applied Optics, Vol.37, No.26, September 1998, page 6227

SUMMARY OF INVENTION

Problems to be Solved by the Invention

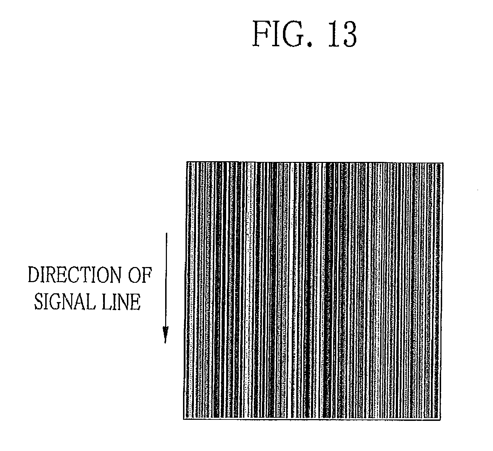

X-ray image detectors for use in X-ray imaging systems employ a TFT method, an optical switching method (optical reading type), or the like. Any of these X-ray image detectors has a configuration in which pixels are arranged in two-dimensions. The pixels arranged in one direction outputs signal charge through a single signal line (read line), and a pixel value is obtained through a detection circuit, for example, an integrating amplifier, and an A/D converter. For this reason, the same amount of noise is added to each pixel value of the pixel group connected to the single signal line due to offset and linearity (linearity of the pixel value relative to voltage) caused by differences in properties of the signal line, the detection circuit, the A/D converter, and the like. Consequently, as shown in Fig. 13, streak noise appears, in a direction of the signal line, in the image obtained with the X-ray image detector. This results in unevenness in the image.
The streak-like unevenness also appears in a differential phase image, which in result deteriorates image quality of a phase contrast image. However, conventional X-ray imaging systems are not provided with a countermeasure to prevent the unevenness in the image.
An object of the present invention is to provide a radiation imaging system for reducing deterioration in image quality of a phase contrast image due to streak noise caused in a direction corresponding to a signal line of an X-ray image detector, and an image processing method for use in a radiation imaging system.

Means for Solving the Problems

To achieve the above objects, a radiation imaging system of the present invention is provided with a first grating, an intensity modulator, a radiation image detector, a differential phase image producing section, and a phase contrast image producing section. The first grating transmits radiation emitted from a radiation source and forms a first periodic pattern image. The intensity modulator provides intensity modulation to the first periodic pattern image to form a second periodic pattern image. The radiation image detector has pixels arranged in two dimensions in a grating line direction of the first grating and an orthogonal direction orthogonal to the grating line direction. A group of pixels arranged in the orthogonal direction are connected to a single signal line that reads out charge. The radiation image detector detects the second periodic pattern image to produce image data. The differential phase image producing section produces a differential phase image based on the image data. The phase contrast image producing section integrates the differential phase image in a direction corresponding to the signal line to produce a phase contrast image.
The intensity modulator provides the intensity modulation to the first periodic pattern image at relative positions out of phase with each other to produce the second periodic pattern images. The radiation image detector detects the second periodic pattern images to produce the respective pieces of image data. The differential phase image producing section calculates a phase shift value of an intensity modulation signal, representing intensity changes of pixel data corresponding to the relative positions, based on the pieces of image data to produce the differential phase image.
The radiation image detector is a TFT type radiation image detector that reads out charge, generated by the radiation in each pixel, to the signal line through a TFT.
The intensity modulator moves one of the first grating and a second grating, which has a periodic pattern in the same direction as a periodic pattern of the first periodic pattern image, in parallel with the signal line at a predetermined pitch.
Each of the first and second gratings is an absorption-type grating. The first grating linearly projects the radiation, from the radiation source, as the first periodic pattern image onto the second grating.

The first grating is a phase-type grating. The first grating may form the radiation, from the radiation source, into the first periodic pattern image due to Talbot effect. The first periodic pattern image is formed at a position of the second grating.

It is preferable that the radiation image detector is an optical reading type and also serves as the intensity modulator. The radiation image detector records the first periodic pattern image as an electrostatic latent image and scans the recorded electrostatic latent image with reading beams to detect the second periodic pattern image, and reads out the second periodic pattern image as the image data.

The radiation image detector is scanned with the reading beams at a pitch shorter than a period of the first periodic pattern image in a direction of a periodic pattern of the first periodic pattern image.

It is preferable that the radiation imaging system further includes a source grating on an emission side of the radiation source.

It is preferable that the radiation imaging system further includes a holder for fixing the radiation image detector in a detachable manner.

It is preferable that the radiation imaging system further includes means for preventing incorrect attachment to prevent the radiation image detector from being attached to the holder in a direction other than a direction in which a signal line direction is orthogonal to the grating line direction.

It is preferable that the radiation imaging system further includes means for prohibiting erroneous exposure to prohibit radiation exposure from the radiation source when the radiation image detector is attached to the holder in a direction other than a direction in which a signal line direction is orthogonal to the grating line direction.

An image processing method of the present invention for use in a radiation imaging system which comprises a first grating, an intensity modulator, and a radiation image detector. The first grating transmits radiation from a radiation source to form a first periodic pattern image. The intensity modulator provides intensity modulation to the first periodic pattern image to form a second periodic pattern image. The radiation image detector has pixels arranged in two-dimensions in a grating line direction of the first grating and an orthogonal direction orthogonal to the grating line direction. A group of the pixels arranged in the orthogonal direction is connected to a single signal line that reads out charge. The radiation image detector detects the second periodic pattern image to produce image data. The image processing method of the present invention has a step for producing a differential phase image based on the image data and a step for integrating the differential phase image in a direction corresponding to the signal line to produce a phase contrast image.

Effect of the Invention

In the present invention, the phase contrast image is produced by integrating the differential phase image, produced by the differential phase image producing section, in the direction corresponding to the signal line that reads out the charge from the radiation image detector. Thereby, deterioration in image quality of the phase contrast image, due to streak noise in the direction corresponding to the signal line, is reduced.

BRIEF DESCRIPTION OF DRAWINGS

Fig. 1 is a schematic view illustrating a configuration of an X-ray imaging system according to a first embodiment of the present invention.
Fig. 2 is a block diagram illustrating a configuration of a flat panel detector (FPD).
Fig. 3 is a perspective view illustrating the FPD and a holder.
Fig. 4 is an explanatory view illustrating a configuration of first and second absorption-type gratings.
Fig. 5 is an explanatory view illustrating a fringe scanning method.
Fig. 6 is a graph illustrating an intensity modulation signal in the presence and absence of a subject.
Fig. 7 is a perspective view illustrating an FPD and a holder according to a second embodiment of the present invention.
Fig. 8 is a perspective view illustrating a multi-slit used in a third embodiment of the present invention.
Fig. 9 is a schematic view illustrating a configuration of an FPD of a fourth embodiment of the present invention.
Fig. 10 is an explanatory view illustrating a configuration of an FPD according to a sixth embodiment of the present invention.
Fig. 11A is an explanatory view describing imaging operation of the FPD according to the sixth embodiment of the present invention.
Fig. 11B is an explanatory view describing imaging operation of the FPD according to the sixth embodiment of the present invention.
Fig. 12 is an explanatory view describing readout processing of the FPD of the sixth embodiment of the present invention.

Fig. 13 illustrates an example of image data obtained with the FPD.

MODE FOR CARRYING OUT THE INVENTION

(First embodiment)

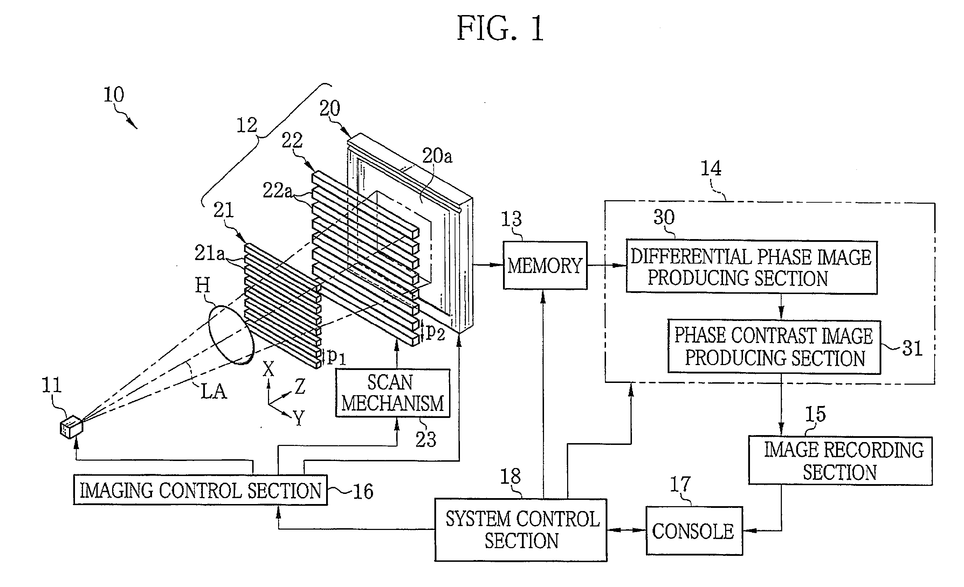

In Fig. 1, an X-ray imaging system 10 according to a first embodiment of the present invention is composed of an X-ray source 11, an imaging unit 12, a memory 13, an image processing section 14, an image recording section 15, an imaging control section 16, a console 17, and a system control section 18. The X-ray source 11 irradiates a subject H with X-rays. The imaging unit 12 is disposed to face the X-ray source 11, and detects the X-rays, emitted from the X-ray source 11 and passed through the subject H, to produce image data. The memory 13 stores the image data read out from the imaging unit 12. The image processing section 14 performs image processing of pieces of image data, stored in the memory 13, to produce a phase contrast image. The image recording section 15 records the phase contrast image produced by the image processing section 14. The imaging control section 16 controls the X-ray source 11 and the imaging unit 12. The console 17 is provided with an operation unit and a monitor. The system control section 18 controls the whole X-ray imaging system 10 based on an operation signal inputted from the console 17.

The X-ray source 11 is composed of a high voltage generator, an X-ray tube, a collimator (all not shown), and the like, and irradiates the subject H with the X-rays based on control of the imaging control section 16. For example, the X-ray tube is a rotating anode type, and releases electron beams from a filament in accordance with voltage applied from the high voltage generator. The X-ray tube generates the X-rays when the anode rotating at a predetermined speed is struck by the electron beams. The anode is rotated to reduce deterioration at a spot bombarded by the electron beams. The spot on the anode struck by the electron beams is an X-ray focal point where the X-rays are generated. The collimator restricts an X-ray field of the X-rays emitted from the X-ray tube so as to block the X-rays not directed toward a region of examination of the subject H.

The imaging unit 12 is provided with a flat panel detector (FPD) 20 composed of a semiconductor circuit, and a first absorption-type grating 21 and a second absorption-type grating 22. The first absorption-type grating 21 and a second absorption-type grating 22 are used to detect a phase shift (angular change) of the X-rays, caused by the subject H, so as to perform phase imaging. The FPD 20 is disposed such that its detection surface 20a is orthogonal to a direction (hereinafter referred to as the Z direction) along an optical axis LA of the X-rays emitted from the X-ray source 11.

The first absorption-type grating 21 has a plurality of X-ray shielding portions (high X-ray absorption portions) 21a extending in a direction (hereinafter referred to as the Y direction) in a plane orthogonal the Z direction and arranged at a predetermined pitch $p_1$ in a direction (hereinafter referred to as the X direction) orthogonal to the Z and Y directions. In a similar manner, the second absorption-type grating 22 has a plurality of X-ray shielding portions (high X-ray absorption portions) 22a extending in the Y direction and arranged at a predetermined pitch $p_2$ in the X direction. Metal with high X-ray absorption properties is preferable as a material of the X-ray shielding portions 21a and 22a. For example, gold (Au) and platinum (Pt) are preferable.

The imaging unit 12 is provided with a scan mechanism 23 that translationally moves the second absorption-type grating 22 in a direction (X direction) orthogonal to a direction (Y direction) of a grating line so as to change a position of the second absorption-type grating 22 relative to that of the first absorption-type grating 21. The scan mechanism 23 is composed of an actuator such as a piezoelectric element. The scan mechanism 23 is driven and controlled by the imaging control section 16 during fringe scanning which will be described below. The memory 13 stores the image data obtained from the imaging unit 12 in respective scanning steps of the fringe scanning, which will be detailed below. Note that the second absorption-type grating 22 and the scan mechanism 23 constitute an intensity modulator.

The image processing section 14 is provided with a differential phase image producing section 30 and a phase contrast image producing section 31. The differential phase image producing section 30 produces a differential phase image based on the pieces of image data captured with the imaging unit 12 in the respective scanning steps in the fringe scanning carried out by the scan mechanism 23 and stored in the memory 13. The phase contrast image producing section 31 integrates the differential phase image, produced by the differential phase image producing section 30, in a scan direction (X direction) to produce the phase contrast image. The phase contrast image, produced by the phase contrast image producing section 31, is recorded in the image recording section 15, and then outputted to the console 17 and displayed on a monitor (not shown).

In addition to the monitor, the console 17 is provided with an input device (not shown) with which an operator inputs an imaging instruction and a content of the instruction. For example, a switch, a touch panel, a mouse, a keyboard, or the like is used as the input device. X-ray imaging conditions such as a tube voltage of the X-ray tube and X-ray exposure time, and imaging timing are inputted using the input device. The monitor is composed of an LCD or a CRT display, and displays the phase contrast image and text such as the X-ray imaging conditions.

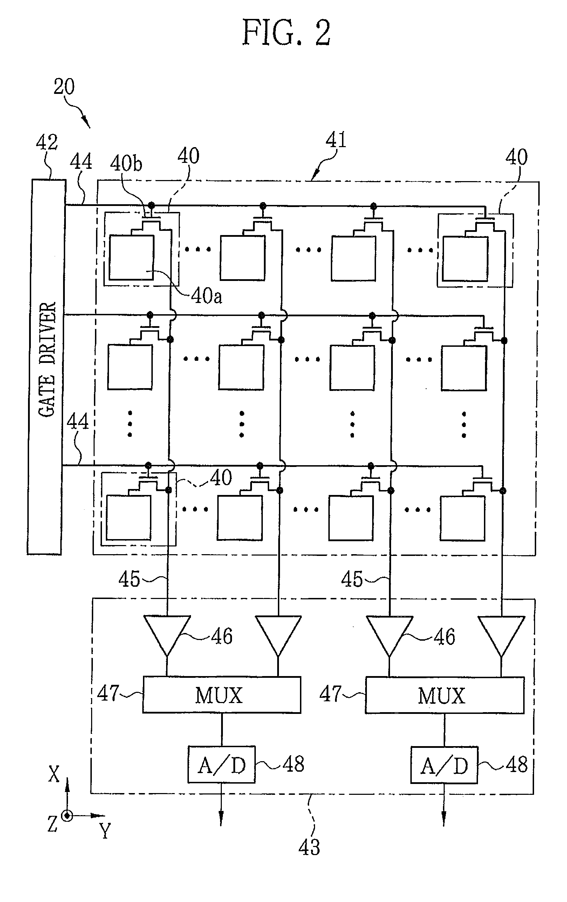

In Fig. 2, the FPD 20 is composed of an image receiving section 41, a gate driver 42, and a readout circuit 43.

The image receiving section 41 is composed of an active matrix substrate and a plurality of pixels 40 arranged on the active matrix substrate in two-dimensions in the X and Y directions. The pixels 40 convert the X-rays into charge and store the charge. The gate driver 42 controls readout timing of the charge from the pixels 40. The readout circuit 43 reads out the charge from the pixels 40 and converts the charge into digital image data and outputs the digital image data.

The pixel 40 is provided with a pixel electrode 40a that collects charge produced by conversion of the X-rays through an X-ray conversion layer (not shown), for example, amorphous selenium, and a thin film transistor (TFT) 40b, being a switching element. A gate electrode of the TFT 40b is connected to a gate scanning line 44, and one of a source electrode and a drain electrode of the TFT 40b is connected to a signal line 45 and the other is connected to the pixel electrode 40a. The gate scanning lines 44 and the signal lines 45 are arranged in a lattice pattern. The gate scanning lines 44 are connected to the gate driver 42. The signal lines 45 are connected to the readout circuit 43. An arrangement pitch of the pixels 40 is in the order of 100 $\mu$m in each of the X and Y directions.

The TFTs 40b of the pixels 40 arranged in the Y direction are connected to the single gate scanning line 44. When the gate scanning line 44 is turned on, the charge of the pixels 40 connected to the gate scanning line 44 is outputted through the signal lines 45 and inputted to the readout circuit 43. The gate driver 42 is composed of a shift register and the like, and turns on the gate scanning line 44 sequentially. The pixels 40 arranged in the X direction are connected to the single signal line 45, through which the charge is outputted to the readout circuit 43.

The readout circuit 43 is composed of integrating amplifiers 46, multiplexers (MUX) 47, and A/D converters 48. The integrating amplifiers 46 are connected to the respective signal lines 45. Each integrating amplifier 46 integrates the charge transmitted from the pixels 40 through the signal line 45, and converts the charge into a voltage signal and outputs the voltage signal. Groups of the integrating amplifiers 46 are connected to the respective multiplexers (MUX) 47. The A/D converters 48 are connected to outputs of the respective MUX 47.

The MUX 47 sequentially selects one of the integrating amplifiers 46 connected, and inputs the voltage signal, outputted from the selected integrating amplifier 46, to the A/D converter 48. The A/D converter 48 digitizes the inputted voltage signal and outputs a digital voltage signal.

Note that the above-described X-ray conversion layer may be an indirect conversion type that converts the X-rays into visible light using a scintillator (not shown) formed from gadolinium oxide ($Gd_2O_3$), cesium iodide (CsI), or the like, and then converts the visible light into charge using a photodiode (not shown). In this embodiment, the FPD 20 is composed of a TFT panel. Alternatively, the FPD 20 can be composed of a solid-state imaging device such as a CCD sensor or a CMOS sensor.

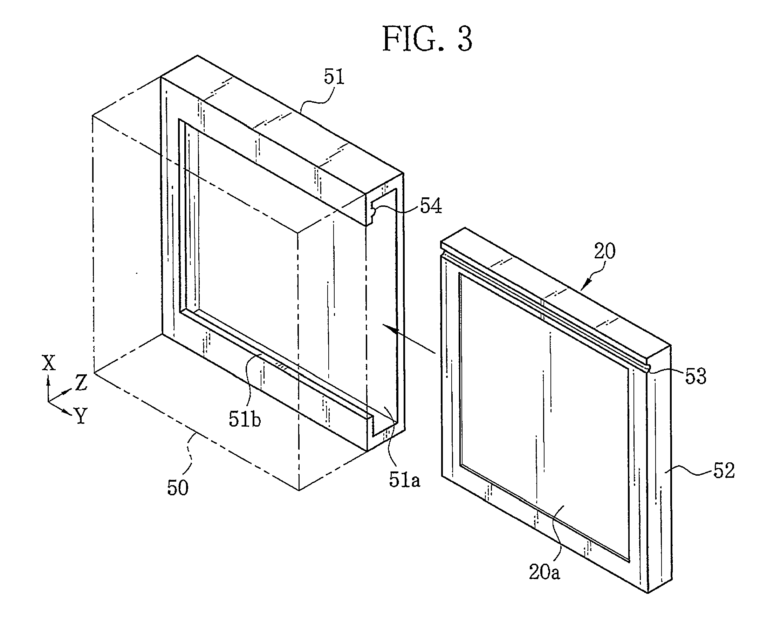

In Fig. 3, the FPD 20 is configured to be fixable to and detachable from a holder 51 formed integrally with a grating module 50. The grating module 50 accommodates the first and second absorption-type gratings 21 and 22. The FPD 20 is provided with a frame 52 that covers edges of the detection surface 20a. The holder 51 is provided with a side opening 51a on one side of the holder 51 and a front opening 51b. The side opening 51a allows insertion of the FPD 20. The front opening 51b exposes the detection surface 20a of the FPD 20 when the FPD 20 is attached to the holder 51.

An upper portion of the frame 52 is formed with a linear groove 53 extending along a top edge of the frame 52. The holder 51 is formed with a linear projection 54 that engages with the groove 53. Because there is only a single pair of the groove 53 and the projection 54, the FPD 20 is attached to the holder 51 only in the direction shown in the drawing. The FPD 20 cannot be inserted into the holder 51 in a direction other than that shown in the drawing because the projection 54 obstructs the insertion.

The FPD 20 is only inserted into the holder 51 in the direction making the signal lines 45 orthogonal to the grating lines of the first and second absorption-type gratings 21 and 22 (in other words, making the signal lines 45 in parallel with a scan direction of the scan mechanism 23). The groove 53 and the projection 54 function as means to prevent incorrect attachment.

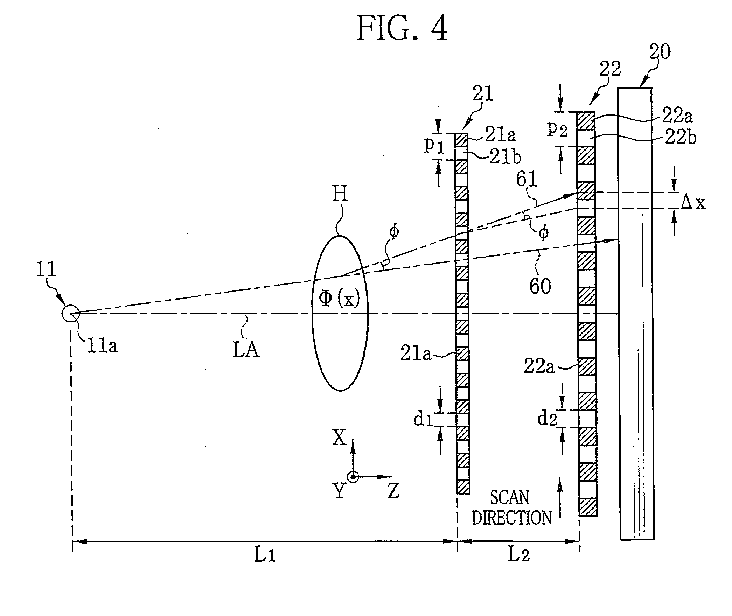

In Fig. 4, the X-ray shielding portions 21a of the first absorption-type grating 21 are arranged in the X direction at a pitch $p_1$ and with a space $d_1$ between each other. A low X-ray absorption portion 21b is provided in each space $d_1$. In a similar manner, the X-ray shielding portions 22a of the second absorption-type grating 22 are arranged in the X direction at a pitch $p_2$ and with a space $d_2$ between each other. A low X-ray absorption portion 22b is provided in each space $d_2$. The first and second absorption-type gratings 21 and 22 do not provide phase shifts, but provides intensity changes to the incident X-rays. The first and second absorption-type gratings 21 and 22 are referred to as amplitude-type gratings. It is preferable that the low X-ray absorption portions 21b and 22b are formed from silicon (Si) or polymer, for example. Alternatively, the low X-ray absorption portions 21b and 22b may be gaps.

The first and the second absorption-type gratings 21 and 22 are configured to project the X-rays, passed through the low X-ray absorption portions 21b and 22b, in a linear (geometrical-optical) manner. To be more specific, each of the spaces $d_1$ and $d_2$ is made sufficiently larger than a peak wavelength of the X-rays emitted from the X-ray source 11. Thereby, most of the emission X-rays pass through the low X-ray absorption portions 21b and 22b in straight lines without diffraction. For example, when the rotating anode of the above-described X-ray tube is made from tungsten and the tube voltage is set to 50 kV, the peak wavelength of the X-rays is approximately 0.4 Å. In this case, most of the X-rays are projected linearly through the low X-ray absorption portions 21b and 22b when the each of the spaces $d_1$ and $d_2$ is in the order of 1 to 10 $\mu$m. Each of the grating pitches $p_1$ and $p_2$ is in the order of 2 to 20 $\mu$m.

Because the X-ray source 11 does not emit parallel beams, but emits the cone-shaped X-ray beams from an X-ray focal point 11a, being a light emission point, a first periodic pattern image (hereinafter referred to as the G1 image) formed by the X-rays passed through the first absorption-type grating 21 is enlarged in proportion to a distance from the X-ray focal point 11a. The grating pitch $p_2$ and the space $d_2$ of the second absorption-type grating 22 are determined such that a pattern of the low X-ray absorption portions 22b substantially coincides with a periodic pattern of bright areas in the G1 image at the position of the second absorption-type grating 22. Namely, the grating pitch $p_2$ and the space $d_2$ are determined to satisfy expressions (1) and (2), where $L_1$ denotes a distance between the X-ray focal point 11a and the first absorption-type grating 21 and $L_2$ denotes a distance between the first absorption-type grating 21 and the second absorption-type grating 22.

$$p_2 = \frac{L_1 + L_2}{L_1} p_1 \quad \cdots (1)$$

$$d_2 = \frac{L_1 + L_2}{L_1} d_1 \quad \cdots (2)$$

When a Talbot interferometer is used, the distance $L_2$ between the first absorption-type grating 21 and the second absorption-type grating 22 is restricted by a Talbot length. The Talbot length is determined by the grating pitch of the first diffraction grating and the X-ray wavelength. In the imaging unit 12 of this embodiment, however, the first absorption-type grating 21 is configured to project the incident X-rays without causing diffraction. Because the G1 image of the first absorption-type grating 21 is obtained, proportionally, at any position behind the first absorption-type grating 21, the distance $L_2$ can be set irrespective of the Talbot length.

As described above, the imaging unit 12 of this embodiment does not constitute the Talbot interferometer. However, when assuming that the X-rays are diffracted by the first absorption-type grating 21 to produce the Talbot effect, a Talbot length $Z_m$ is represented by an expression (3) using the grating pitch $p_1$ of the first absorption-type grating 21, the grating pitch $p_2$ of the second absorption-type grating 22, the X-ray wavelength (peak wavelength) $\lambda$, and a positive integer m.

$$Z_m = m \frac{p_1 p_2}{\lambda} \quad \cdots (3)$$

In this embodiment, as described above, the distance $L_2$ can be set irrespective of the Talbot length $Z_m$. To reduce the thickness of the imaging unit 12 in the Z direction, the distance $L_2$ is set shorter than a minimum Talbot length $Z_1$ (when m=1). Namely, the distance $L_2$ is set to a value within a range satisfying an expression (4).

$$L_2 < \frac{p_1 p_2}{\lambda} \quad \cdots (4)$$

To produce a periodic pattern image with high contrast, it is preferable that the X-ray shielding portions 21a and 22a completely block (absorb) the X-rays. However, even if the material (gold, platinum, or the like) with high X-ray absorption properties is used, the X-rays passing through the X-ray shielding portions 21a and 22a still exist. To improve the X-ray shielding properties, it is preferable to increase the thickness (in the Z direction) of each of the X-ray shielding portions 21a and 22a (namely, to increase an aspect ratio) as much as possible. For example, it is preferable to block 90% or more of the emission X-rays when the tube voltage of the X-ray tube is 50kV. It is preferable that the thickness of each of the X-ray shielding portions 21a and 22a is in the range of 10 $\mu$m to 200 $\mu$m.

With the use of the above-described first and second absorption-type gratings 21 and 22, the G1 image produced with the first absorption-type grating 21 is partly blocked by the superposition of the second absorption-type grating 22 and thereby subjected to the intensity modulation. Thus, a second periodic pattern (hereinafter referred to as the G2 image) is produced. The G2 image is captured with the FPD 20.

There is a slight difference between a pattern period of the G1 image at the position of the second absorption-type grating 22 and the grating pitch $p_2$ of the second absorption-type grating 22 due to arrangement error or the like. Due to this minute difference, moiré fringes occur in the G2 image. When error occurs in grating arrangement directions of the first and second absorption-type gratings 21 and 22, and the grating arrangement directions of the first and second absorption-type gratings 21 and 22 are not the same, so-called rotational moiré fringes occur in the G2 image. The rotational moiré fringes do not cause any problem when a period of the moiré fringes in the X or Y direction is greater than an arrangement pitch of the pixels 40. If possible, it is preferable to prevent occurrence of moiré fringes. However, the moiré fringes can be used to check an amount of scanning (a distance by which the second absorption-type grating 22 is translationally moved) in the fringe scanning.

When the subject H is disposed between the X-ray source 11 and the first absorption-type grating 21, the G2 image detected by the FPD 20 is modulated by the subject H. An amount of the modulation is in proportion to an angle of the X-rays shifted due to refraction effect of the subject H. The phase contrast image of the subject H is produced by analyzing the G2 image detected by the FPD 20.

Next, a method for analyzing the G2 image is described in principle. Fig. 4 illustrates an X-ray beam refracted in accordance with phase shift distribution $\Phi(x)$ relative to the X direction of the subj ect H by way of example. A numeral 60 denotes a path of an X-ray beam in the absence of the subject H. The X-ray beam traveling along the path 60 passes through the first and the second absorption-type gratings 21 and 22, and then is incident on the FPD 20. When in the presence of the subject H, a numeral 61 denotes a path of the X-ray beam shifted by the refraction of the subject H. The X-ray beam traveling along the path 61 passes through the first absorption-type grating 21, but then is blocked by the X-ray shielding portions 22a of the second absorption-type grating 22.

The phase shift distribution $\Phi(x)$ of the subject H is represented by an expression (5), where n (x, z) denotes refractive index distribution of the subject H, and z denotes a direction in which the X-rays are transmitted.

$$\Phi(x) = \frac{2\pi}{\lambda} \iint \left[ 1 - n(x,\ z) \right] dz \quad \cdots (5)$$

The G1 image, projected from the first absorption-type grating 21 onto the position of the second absorption-type grating 22, is displaced in the X direction by an amount corresponding to a refraction angle $\varphi$ at the subject. A displacement amount $\Delta x$ is represented approximately by an expression (6) on the basis that the refraction angle $\varphi(x)$ of the X-rays is minute.

$$\Delta x \approx L_2 \phi \quad \cdots (6)$$

The refraction angle $\varphi$ is represented by an expression (7) using the X-ray wavelength $\lambda$ and the phase shift distribution $\Phi(x)$ of the subject H.

$$\phi = \frac{\lambda}{2\pi} \frac{\partial \Phi(x)}{\partial x} \quad \cdots (7)$$

As described above, the displacement amount $\Delta x$ of the G1 image, caused by the X-ray refraction at the subject H, relates to the phase shift distribution $\Phi(x)$ of the subject H. The displacement amount $\Delta x$ relates to a phase shift value $\psi$ of the intensity modulation signal of each of the pixels 40 detected by the FPD 20 (that is, a phase shift value of the intensity modulation signal of each of the pixels 40 between in the presence and absence of the subject), in a way that they satisfy an expression (8).

$$\psi = \frac{2\pi L_2}{p_2}\phi = \frac{2\pi}{p_2}\Delta x \quad \cdots (8)$$

By obtaining the phase shift value $\psi$ of the intensity modulation signal of each of the pixels 40, the refraction angle $\varphi$ is calculated using the expression (8) and a differential value of the phase shift distribution $\Phi(x)$ is calculated using the expression (7). By integrating the differential value relative to x, the phase shift distribution $\Phi(x)$ of the subject H, that is, the phase contrast image of the subject H is produced.

In the fringe scanning method, the image is captured with one of the first and the second absorption-type gratings 21 and 22 translationally moved relative to the other in the X direction (namely, the image is captured with the phases of the grating periods of the first and second absorption-type gratings 21 and 22 changed). In this embodiment, the above-described scan mechanism 23 moves the second absorption-type grating 22. The moiré fringes in the G2 image move with the movement of the second absorption-type grating 22, and return to its original position when the translational distance (an amount of movement in the X direction) reaches one grating period (the grating pitch $p_2$) of the second absorption-type grating 22 (namely, when the phase shift reaches 2n). Thus, the G2 images are captured using the FPD 20 with the second absorption-type grating 22 moved by an integral fraction of the grating pitch $p_2$. The intensity modulation signal of each pixel is obtained from the pieces of image data obtained from the image capture. The intensity modulation signal is subjected to arithmetic processing in the differential phase image producing section 30 in the above-described image processing section 14. Thereby, the phase shift value $\psi$ of the intensity modulation signal of each pixel is obtained. Two-dimensional distribution of the phase shift value $\psi$ corresponds to the differential phase image.

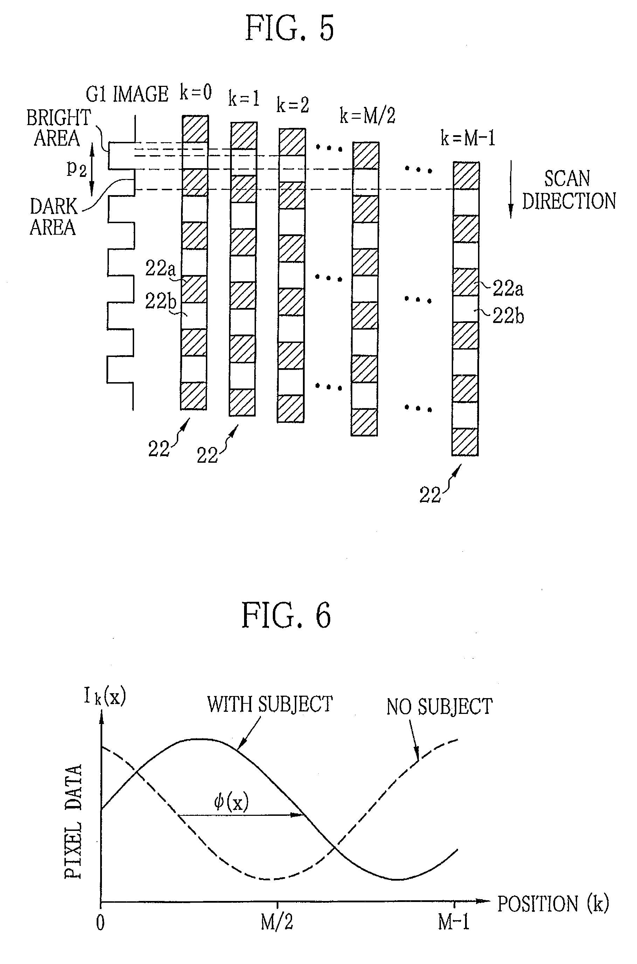

Fig. 5 schematically illustrates the second absorption-type grating 22 moved at a scanning pitch $(p_2/M)$ obtained by dividing the grating pitch $p_2$ by a number M (an integer greater than or equal to 2). The scan mechanism 23 translationally moves the second absorption-type grating 22 to each of M number of scan positions (k=0, 1, 2,..., M-1). Note that, in the drawing, an initial position of the second absorption-type grating 22 is a position (k=0) where dark areas of the G1 image at the position of the second absorption-type grating 22 substantially coincide with the X-ray shielding portions 22a in the absence of the subject H. Alternatively, the initial position may be any one of the positions (k=0, 1, 2,..., M-1).

First, at the position (k=0), mainly, a component (non-refractive component) of the X-rays not refracted by the subject H passes through the second absorption-type grating 22. Next, as the second absorption-type grating 22 is moved to each of the positions (k=1, 2,...) sequentially, the non-refractive component decreases while a component (refractive component) of the X-rays refracted by the subject H increases in the X-rays passing through the second absorption-type grating 22. At the position (k=M/2), mainly (and substantially only) the refractive component passes through the second absorption-type grating 22. At the positions subsequent to the position (k=M/2), on the contrary, the refractive component decreases while the non-refractive component increases in the X-rays passing through the second absorption-type grating 22.

After the image is captured and the image data is produced at each of the positions (k=0, 1, 2, ..., M-1), the M number of pixel data are obtained per pixel 40. Hereinafter, a method for calculating the phase shift value $\psi$, of the intensity modulation signal of each of the pixels 40, from the M number of pixel data is described. Pixel data $I_k(x)$ of each of the pixels 40 at the time the second absorption-type grating 22 is positioned at a position k is generally represented by an expression (9).

$$I_k(x) = A_0 + \sum_{n>0} A_n \exp\left[2\pi i \frac{n}{p_2}\left\{L_2\phi(x) + \frac{kp_2}{M}\right\}\right] \quad \cdots (9)$$

Here x denotes a coordinate of the pixel in the X direction, $A_0$ denotes the intensity of the incident X-rays, $A_n$ denotes a value corresponding to the contrast of the intensity modulation signal, n denotes a positive integer, and i denotes an imaginary unit. The $\varphi(x)$ denotes the refraction angle $\varphi$ expressed as a function of the coordinate x of the pixel 40.

When a relational expression (10) is applied, the refraction angle φ(x) is represented by an expression (11).

$$\sum_{k=0}^{M-1} \exp\left(-2\pi i \frac{k}{M}\right) = 0 \quad \cdots (10)$$

$$\phi(x) = \frac{p_2}{2\pi L_2} \arg\left[\sum_{k=0}^{M-1} I_k(x) \exp\left(-2\pi i \frac{k}{M}\right)\right] \quad \cdots (11)$$

Here, arg[ ] represents extraction of an argument and corresponds to the phase shift value ψ(x). The refraction angle φ(x) is obtained by calculating the phase shift value ψ(x) based on the expression (11) using the intensity modulation signal. The intensity modulation signal is represented by the M numbers of the pixel data obtained from each of the pixels 40. Note that the refraction angle φ (x) is in proportion to the phase shift value ψ(x) as shown by the expression (8), so that both the refraction angle φ(x) and the phase shift value ψ(x) are physical quantities corresponding to the differential value of the phase shift distribution Φ(x). By integrating the refraction angle φ(x) and the phase shift value ψ(x) in the X direction, a physical quantity corresponding to the phase shift distribution Φ(x) is obtained.

To be more specific, as shown in Fig. 6, the intensity modulation signal varies periodically with a period of the grating pitch $p_2$ relative to the position k of the second absorption-type grating 22. A broken line in the drawing denotes the intensity modulation signal in the absence of the subject H. A solid line in the drawing denotes the intensity modulation signal in the presence of the subject H. A phase difference between the waveforms of the broken line and the solid line corresponds to the phase shift value ψ(x).

In the above description, the y coordinate of the pixel 40 in the Y direction is not considered. By performing the calculation similar to the above, a two-dimensional phase shift value ψ(x, y) relative to the X and Y directions is obtained. The phase shift distribution Φ(x, y) of the subject H is calculated using an integration process represented by an expression (12) using the phase shift value ψ(x, y).

$$\Phi(x, y) = \frac{p_2}{\lambda L_2} \int_0^x (x, y)dx \quad \cdots (12)$$

Next, operation of the above-configured X-ray imaging system 10 is described. When the FPD 20 is attached to the holder 51 and when the start of the imaging is instructed through the console 17, the system control section 18 controls each section. The scanning of the second absorption-type grating 22 using the scan mechanism 23, the X-ray exposure with the X-ray source, and the detection operation with the FPD 20 are performed. As a result of the fringe scanning, the phase contrast image producing section 31 produces the differential phase image ψ(x, y) based on the pieces of image data stored in the memory 13. The phase contrast image producing section 31 performs the integration process based on the expression (12), and produces the phase contrast image. The phase contrast image is recorded in the image recording section 15, and then outputted to the console 17 and displayed on the monitor.

As shown in Fig. 13, streak noise in a direction corresponding to the signal line 45 occurs in each image data stored in the memory 13 due to a difference in properties between the signal lines 45, a difference in properties between the integrating amplifiers 46 connected to the respective signal lines 45, and a difference in properties between the A/D converters 48. The streak noise also appears in the differential phase image ψ(x, y) in the X direction.

To prevent the streak noise, in this embodiment, the PFD 20 is held by the holder 51 such that the signal lines 45 are in parallel with the scan direction of the scan mechanism 23. Thereby, the direction (X direction) of the integration of the differential phase image ψ(x, y) performed by the phase contrast image producing section 31 coincides with the direction of the streak noise. As shown by the expression (12), the differential phase image ψ (x, y) is integrated in the X direction from the origin 0 to the coordinate x. In this embodiment, the amount of noise is substantially uniform in the integration direction and a change in the amount of noise is small, so that the change in the amount of noise is not likely to be reflected to the result of the integration. Thus, the deterioration in the image quality in the phase contrast image is reduced. When the integration direction is orthogonal to the direction of the streak noise, the amounts of noise vary drastically in the integration direction. The change in the amount of noise strongly affects the result of the integration. As a result, the image quality of the phase contrast image deteriorates.

(Second embodiment)

In the first embodiment, the groove 53 provided in the FPD 20 and the projection 54 provided in the holder 51 allow the FPD 20 to be engaged with the holder 51 in only one direction. Thereby, the incorrect attachment of the FPD 20 is prevented. Alternatively, the FPD 20 may be configured to detect whether the FPD 20 is attached in the correct direction, and the exposure is allowed only when the FPD 20 is attached in the correct direction.

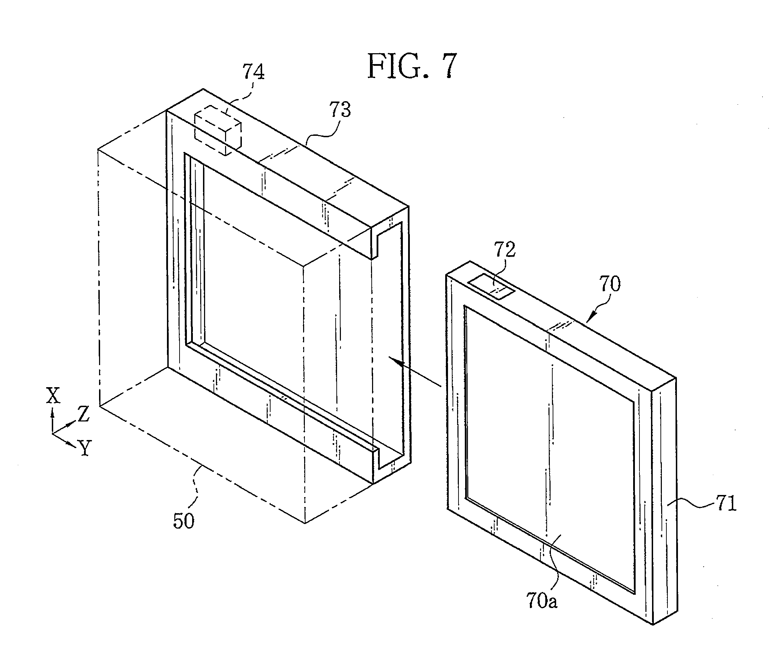

As shown in Fig. 7, in a second embodiment of the present invention, an FPD 70 is provided with an information provision section 72 to which information is provided in a form of a bar code, an IC tag, or the like. The information provision section 72 is provided in a position on a frame 71 covering edges of a detection surface 70a. A holder 73, to which the FPD 70 is attached, is provided with an information reading section 74, for example, a bar code reader or an IC tag reader. The information reading section 74 is provided in a position close to the information provision section 72 of the FPD 70 inserted into the holder 73. Note that a configuration of the FPD 70 is the same as that of the FPD 20 of the first embodiment except that the frame 71 has the information provision section 72 instead of the groove. The configuration of the holder 73 is the same as that of the holder 51 of the first embodiment except that the holder 73 has the information reading section 74 instead of the projection.

In this embodiment, the system control section 18 monitors a read signal from the information reading section 74, and permits the X-ray exposure from the X-ray source 11 only when the information of the information provision section 72 of the FPD 70 is readable, namely, only when the FPD 70 is attached to the holder 73 in the correct direction. Thereby, the image is captured only when the signal lines 45 of the FPD 70 are in parallel with the scan direction of the scan mechanism 23. Thus, the information provision section 72 and the information reading section 74 function as means for prohibiting erroneous exposure. Note that the information provision section 72 and the information reading section 74 may be composed of an electrical contact and a switch.

(Third embodiment)

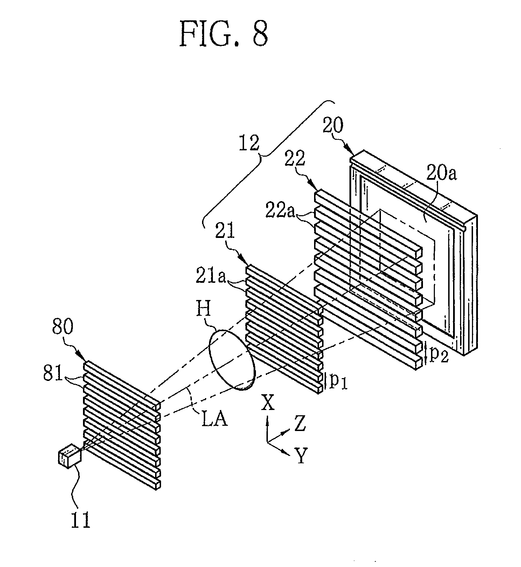

In the first embodiment, when the distance between the X-ray source 11 and the FPD 20 is elongated, the image quality of the phase contrast image may be deteriorated by influence of blur in the G1 image due to the focal point size (generally in the order of 0.1 mm to 1 mm) of the X-ray focal point 11a. In a third embodiment of the present invention, as shown in Fig. 8, a multi-slit (source grating) 80 is disposed on the emission side of the X-ray source 11. The X-ray imaging system of the third embodiment is the same as that of the first embodiment except for the multi-slit 80.

The multi-slit 80 is an absorption-type grating having a configuration similar to those of the first and second absorption-type gratings 21 and 22. The multi-slit 80 has a plurality of X-ray shielding portions 81 extending in the Y direction and arranged periodically in the X direction. The multi-slit 80 partly blocks the X-rays from the X-ray source 11 to reduce the effective focal point size in the X direction. The multi-slit forms a plurality of point light sources (dispersed light sources) to reduce the blur in the G1 image. Note that, in a manner similar to the above, a low X-ray absorption portion (not shown) is provided between the X-ray shielding portions 81 adjacent in the X direction.

(Fourth embodiment)

In the first embodiment, the first absorption-type grating 21 is configured to linearly project the X-rays, passed through the low X-ray absorption portions 21b, as the G1 image. The present invention is not limited to this configuration. The first absorption-type grating 21 can be configured to diffract the X-rays so as to produce the so-called Talbot effect as disclosed in Japanese Patent No. 4445397. In a fourth embodiment of the present invention, the first absorption-type grating 21 is a diffraction grating and the distance between the first and second absorption-type gratings 21 and 22 is set to the Talbot length to constitute a Talbot interferometer. In this embodiment, the G1 image (self image) of the first grating 21 produced due to the Talbot effect is formed at the position of the second absorption-type grating 22.

In this embodiment, the first absorption-type grating 21 may be a phase-type grating (phase-type diffraction grating). In this case, the thickness and the material are determined such that a phase difference of "n" or "0.5n" of the X-rays occurs between the high X-ray absorption portion 21a and the low X-ray absorption portion 21b.

Note that, in each of the above embodiments, the subject H is disposed between the X-ray source 11 and the first absorption-type grating 21. Alternatively, the subject H may be disposed between the first absorption-type grating 21 and the second absorption-type grating 22. The phase contrast image can be produced in a manner similar to the above.

(Fifth embodiment)

In each of the above embodiments, the second absorption-type grating 22 is provided separately from the FPD 20. The second absorption-type grating 22 can be omitted by the use of an X-ray image detector having a configuration disclosed in Japanese Patent Laid-Open Publication No. 2009-133823.

The X-ray image detector of this embodiment is a direct-conversion type X-ray image detector provided with a conversion layer for converting the X-rays into the charge, and a charge collection electrode for collecting the charge converted in the conversion layer. The charge collection electrode of each pixel is composed of linear electrode groups each having linear electrodes. The linear electrodes are arranged at a predetermined period and electrically connected to each other. The linear electrode groups are arranged out of phase with each other. In this embodiment, the charge collection electrode constitutes the intensity modulator.

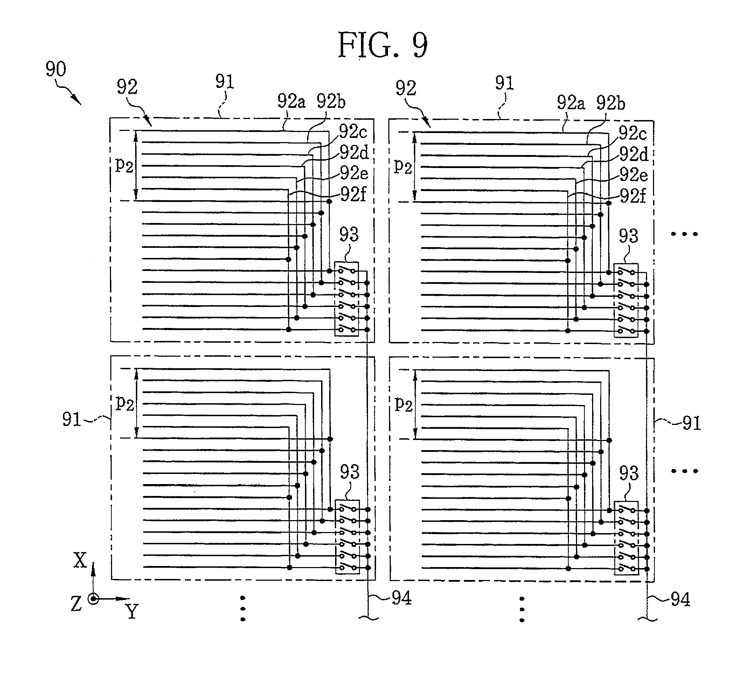

In Fig. 9, an FPD 90 of this embodiment has pixels 91 arranged at a predetermined pitch in two dimensions in the X and Y directions. Each of the pixels 91 is formed with a charge collection electrode 92 for collecting the charge converted by the conversion layer that converts the X-rays into the charge. The charge collection electrode 92 is composed of first to sixth linear electrode groups 92a to 92f. A phase of an arrangement period of the linear electrodes in each of the linear electrode groups is shifted by n/3. To be more specific, when the phase of the first linear electrode group 92a is 0, the phase of the second linear electrode group 92b is n/3; the phase of the third linear electrode group 92c is 2n/3; the phase of the fourth linear electrode group 92d is n; the phase of the fifth linear electrode group 92e is $4\pi/3$; the phase of the sixth linear electrode group 92f is $5\pi/3$.

Each of the pixels 91 is provided with a switch group 93 that reads out the charge collected by the charge collection electrode 92. The switch group 93 is composed of a TFT switch provided to each of the first to sixth linear electrode groups 92a to 92f. The charge collected by each of the first to sixth linear electrode groups 92a to 92f is read out separately by controlling the switch group 93. Thereby, the charge is transmitted to an integrating amplifier (not shown) through a signal line 94. The pixels 91 arranged in the X direction are connected to the single signal line 94 through the respective switch groups 93.

In this embodiment, with the use of the FPD 90, 6 different G2 images that are out of phase with each other are detected by the single image capture. Based on the pieces of image data corresponding to the respective 6 different G2 images, the phase contrast image is produced. Other components are similar to those in the first embodiment, so that the descriptions thereof are omitted.

In this embodiment, the imaging unit 12 omits the second absorption-type grating 22, which reduces cost and enables further reduction in the thickness. In this embodiment, the intensity of the G2 images is modulated at different phases by the single image capture. This makes physical scanning for the fringe scanning unnecessary and omits the scan mechanism 23. Note that a charge collection electrode of another configuration disclosed in the Japanese Patent Laid-Open Publication 2009-133823 can be used instead of the charge collection electrode 92 of the above configuration.

(Sixth embodiment)

As another embodiment omitting the second absorption-type grating 22, an X-ray image detector of an optical switching method (optical readout method) can be used. In a sixth embodiment of the present invention, an X-ray image detector of the following configuration is used.

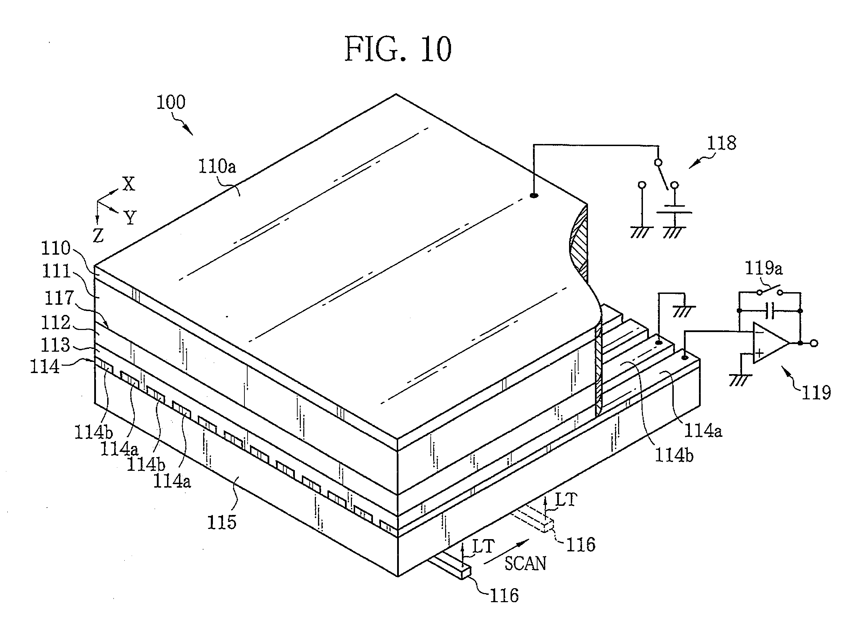

In Fig. 10, an FPD 100 is composed of a first electrode layer 110, a recording photoconductive layer 111, a charge transport layer 112, a reading photoconductive layer 113, a second electrode layer 114, a glass substrate 115, and a line light source 116. The first electrode layer 110 transmits the X-rays. The recording photoconductive layer 111 generates charge pairs (electron-hole pairs) upon incidence of the X-rays passed through the first electrode layer 110. The charge transport layer 112 acts as an insulator to the charge of a polarity and as a conductor to the charge of the opposite polarity. The reading photoconductive layer 113 generates the charge pairs (electron-hole pairs) upon incidence of reading beams LT. The line light source 116 generates the linear reading beams LT. A capacitor portion 117 is formed at an interface between the recording photoconductive layer 111 and the charge transport layer 112. The capacitor portion 117 accumulates one of the positive charge and the negative charge of the charge pairs generated in the recording photoconductive layer 111. Note that the layers are formed in the above-mentioned order with the second electrode layer 114 on the glass substrate 115.

The first electrode layer 110 is gold (Au) with the thickness of approximately 100 nm. The recording photoconductive layer 111 is amorphous selenium (a-Se) with the thickness of approximately 10 $\mu$m to 1500 $\mu$m. It is preferable that the charge transport layer 112 has a large difference between mobility of charge in the first electrode layer 110 and mobility of charge of reverse polarity (for example, $10^2$ or more, preferably $10^3$ or more). The reading photoconductive layer 113 is a-Se with the thickness of approximately 5 $\mu$m to 20 $\mu$m.

The second electrode layer 114 has a plurality of transparent linear electrodes 114a that transmit the reading beams LT and a plurality of light-shielding linear electrodes 114b that block the reading beams LT. The transparent linear electrodes 114a and the light-shielding linear electrodes 114b extend linearly in the X direction from end to end of a detection surface. The transparent linear electrodes 114a and the light-shielding linear electrodes 114b are arranged alternately and in parallel with each other in the Y direction at regular intervals.

The transparent linear electrode 114a is made from a material which has conductivity and transmits the reading beams LT. The transparent linear electrode 114a is made from, for example, ITO, IZO, or IDIXO with the thickness in the order of 100 nm to 200 nm.

The light-shielding linear electrode 114b is made from a material which has conductivity and blocks the reading beams LT. The light-shielding linear electrode 114b transmits erase light that erases charge remaining in the capacitor portion 117 after a reading process which will be described below. It is preferable that the light-shielding linear electrode 114b is composed of a combination of a color filter and the above-described transparent conductive material. The color filter blocks the reading beams LT but transmits the erase light. The thickness of the transparent conductive material is in the order of 100 nm to 200 nm.

The line light source 116 extends in the Y direction from end to end of the detection surface and orthogonal to every transparent linear electrode 114a. The line light source 116 is disposed below the glass substrate 115. The line light source 116 delivers the reading beams LT to the reading photoconductive layer 113 through the glass substrate 115 and the second electrode layer 114 during the reading process which will be described below. The line light source 116 is configured to translationally move in the X direction using a moving mechanism (not shown). Emission of the reading beams LT and the translational movement are controlled by the above-described imaging control section 16.

The FPD 100 is provided with a power supply circuit 118 that supplies a negative voltage or grounding potential to the first electrode layer 110 in accordance with the control of the imaging control section 16. The power supply circuit 118 supplies the negative voltage to the first electrode layer 110 when an X-ray image is recorded. The power supply circuit 118 supplies the grounding potential to the first electrode layer 110 in the reading process, which will be detailed below.

In addition, the FPD 100 is provided with integrating amplifiers 119 that reads out the charge, generated in the reading photoconductive layer 113, in the reading process. The integrating amplifiers 119 are connected to the respective transparent linear electrodes 114a. Each of the light-shielding linear electrodes 114b is grounded. Each integrating amplifier 119 integrates the charge and is provided with a reset switch 119a for resetting the integrated charge. The reset switch 119a is controlled by the imaging control section 16.

In the reading process, the line light source 116 is moved (scanned) at a predetermined pitch in the X direction, and emits the reading beams LT at each position (hereinafter referred to as the scan position) to which the line light source 116 is moved. The integrating amplifier 119 reads out the charge, generated by the reading beams LT in the reading photoconductive layer 113, through the transparent linear electrodes 114a. Thereby, the G2 images out of phase with each other are detected. The pitch (hereinafter referred to as the scanning pitch) to move the line light source 116 is sufficiently shorter than a pattern period in the X direction of the G1 image that is formed by the absorption-type grating 21 and projected onto a detection surface (a surface 110a of the first electrode layer 110) of the FPD 100. Note that the arrangement pitch of the transparent linear electrodes 114a in the Y direction and the arrangement pitch of the light-shielding linear electrodes 114b in the Y direction are not particularly limited, but they are preferably similar to the pattern period.

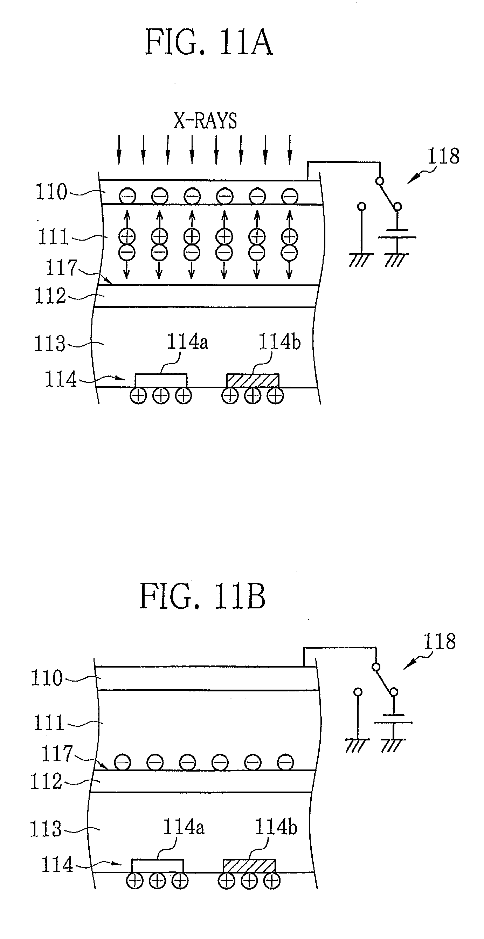

Next, imaging operation of the FPD 100 at each scan position is described. As shown in Fig. 11A, the X-rays emitted toward the FPD 100 pass through the first electrode layer 110 and are incident on the recording photoconductive layer 111. The X-ray emission generates charge pairs in the recording photoconductive layer 111. At this time, the negative voltage is applied to the first electrode layer 110 by the power supply circuit 118 and the first electrode layer 110 is charged with negative charge. Of the charge pairs generated in the recording photoconductive layer 111, the positive charge and the negative charge are bound together in the first electrode layer 110 to cancel each other. As shown in Fig. 11B, the negative charge, being latent image charge (electrostatic latent image), is accumulated in the capacitor portion 117.

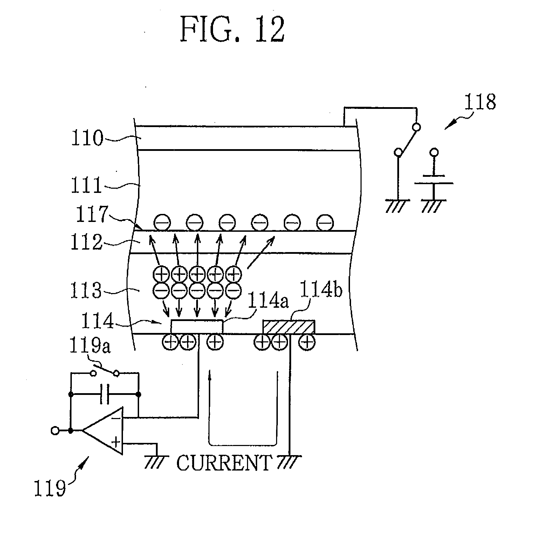

Then, as shown in Fig. 12, the line light source 116 emits the reading beams LT in a state that the first electrode layer 110 is grounded by the power supply circuit 118. The reading beams LT pass through the transparent linear electrodes 114a and are incident on the reading photoconductive layer 113. The positive charge, of the charge pairs generated in the reading photoconductive layer 113, and the latent image charge are bound together in the capacitor portion 117 while the negative charge and the positive charge, charged in the light-shielding linear electrodes 114b, are bound together through the integrating amplifier 119.

Thereafter, the bond between the negative charge generated in the reading photoconductive layer 113 and the positive charge charged in the light-shielding linear electrode 114b allows a current to flow into the integrating amplifier 119. The current is integrated and outputted as a pixel signal. The pixel signal outputted from the integrating amplifier 119 is digitized by an A/D converter (not shown) and sequentially inputted as image data to the memory 13.

In this embodiment, the transparent linear electrodes 114a correspond to the respective signal lines, so that the transparent linear electrodes 114a are in parallel with the scan direction of the fringe scanning (namely, in parallel with a direction in which the differential phase image is integrated).

(Seventh embodiment)

In the above embodiments, the differential phase image is obtained using the fringe scanning method. Alternatively, the differential phase image may be obtained using Fourier transform method disclosed in WO2010/050483. In the Fourier transform method, a piece of image data obtained using the X-ray image detector is subjected to Fourier transform. Thereby a Fourier spectrum of moiré fringes occurred in the image data is obtained. A spectrum corresponding to carrier frequency is separated from the Fourier spectrum, and inverse Fourier transform is performed. Thereby, the differential phase image is produced. The phase contrast image is produced by integrating the differential phase image in a manner similar to the above.

Each of the above-described embodiments can be applied to radiation imaging systems for other uses, for example, for industrial use, in addition to the radiation imaging systems for medical diagnosing. Instead of the X-rays, gamma rays or the like can be used as the radiation.

**Claims**

1. A radiation imaging system comprising:

    a first grating for transmitting radiation emitted from a radiation source and forming a first periodic pattern image;
    an intensity modulator for providing intensity modulation to the first periodic pattern image to form a second periodic pattern image;
    a radiation image detector having pixels arranged in two dimensions in a grating line direction of the first grating and an orthogonal direction orthogonal to the grating line direction, a group of the pixels arranged in the orthogonal direction being connected to a single signal line that reads out charge, the radiation image detector detecting the second periodic pattern image to produce image data;
    a differential phase image producing section for producing a differential phase image based on the image data; and
    a phase contrast image producing section for integrating the differential phase image in a direction corresponding to the signal line to produce a phase contrast image.

2. The radiation imaging system claimed in claim 1, wherein the intensity modulator provides the intensity modulation to the first periodic pattern image at relative positions out of phase with each other to produce the second periodic pattern images, and the radiation image detector detects the second periodic pattern images to produce the respective pieces of image data, and the differential phase image producing section calculates a phase shift value of an intensity modulation signal, representing intensity changes of pixel data corresponding to the relative positions, based on the pieces of image data to produce the differential phase image.

3. The radiation imaging system claimed in claim 1, wherein the radiation image detector is a TFT type radiation image detector that reads out charge, generated by the radiation in each pixel, to the signal line through a TFT.

4. The radiation imaging system claimed in claim 2, wherein the intensity modulator is provided with a second grating and a scan mechanism, and the second grating has a periodic pattern in the same direction as a periodic pattern of the first periodic pattern image, and the scan mechanism moves one of the first and second gratings at a predetermined pitch in a direction parallel with the signal line.

5. The radiation imaging system claimed in claim 4, wherein each of the first and second gratings is an absorption-type grating, and the first grating linearly projects the radiation, from the radiation source, as the first periodic pattern image onto the second grating.

6. The radiation imaging system claimed in claim 4, wherein the first grating is a phase-type grating, and the first grating forms the radiation from the radiation source into the first periodic pattern image due to Talbot effect, and the first periodic pattern image is formed at a position of the second grating.

7. The radiation imaging system claimed in claim 2, wherein the radiation image detector is an optical reading type and also serves as the intensity modulator, and the radiation image detector records the first periodic pattern image as an electrostatic latent image and scans the recorded electrostatic latent image with reading beams to detect the second periodic pattern image, and reads out the second periodic pattern image as the image data.

8. The radiation imaging system claimed in claim 7, wherein the radiation image detector is scanned with the reading beams at a pitch shorter than a period of the first periodic pattern image in a direction of a periodic pattern of the first periodic pattern image.

9. The radiation imaging system claimed in claim 1, further comprising a source grating on an emission side of the radiation source.

10. The radiation imaging system claimed in claim 1, further comprising a holder for fixing the radiation image detector in a detachable manner.

11. The radiation imaging system claimed in claim 10, further comprising means for preventing incorrect attachment, the means preventing the radiation image detector from being attached to the holder in a direction other than a direction in which a signal line direction is orthogonal to the grating line direction.

12. The radiation imaging system claimed in claim 10, further comprising means for prohibiting erroneous exposure, the means prohibiting radiation exposure from the radiation source when the radiation image detector is attached to the holder in a direction other than a direction in which a signal line direction is orthogonal to the grating line direction.

13. An image processing method for use in a radiation imaging system, the radiation imaging system comprising a first grating, an intensity modulator, and a radiation image detector, the first grating transmitting radiation from a radiation source to form a first periodic pattern image, the intensity modulator providing intensity modulation to the first periodic pattern image to form a second periodic pattern image, the radiation image detector having pixels arranged in two-dimensions in a grating line direction of the first grating and an orthogonal direction orthogonal to the grating line direction, a group of the pixels arranged in the orthogonal direction being connected to a single signal line that reads out charge, the radiation image detector detecting the second periodic pattern image to produce image data, the image processing method comprising:

   a step for producing a differential phase image based on the image data; and
   a step for integrating the differential phase image in a direction corresponding to the signal line to produce a phase contrast image.

# FIG. 1

# FIG. 2

# FIG. 3

EP 2 591 727 A1

# FIG. 4

EP 2 591 727 A1

# FIG. 5

# FIG. 6

# FIG. 7

EP 2 591 727 A1

# FIG. 8

FIG. 9

EP 2 591 727 A1

# FIG. 10

EP 2 591 727 A1

# FIG. 11A

# FIG. 11B

# FIG. 12

# FIG. 13

DIRECTION OF
SIGNAL LINE

<div style="text-align:center">**INTERNATIONAL SEARCH REPORT**</div>

| | International application No. |
|---|---|
| | PCT/JP2011/065153 |

| A. CLASSIFICATION OF SUBJECT MATTER |
|---|
| *A61B6/00*(2006.01)i |

According to International Patent Classification (IPC) or to both national classification and IPC

| B. FIELDS SEARCHED |
|---|
| Minimum documentation searched (classification system followed by classification symbols)<br>A61B6/00 |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| A | WO 2008/102654 A1 (Konica Minolta Medical & Graphic, Inc.),<br>28 August 2008 (28.08.2008),<br>paragraphs [0089] to [0122]<br>(Family: none) | 1-13 |
| A | JP 2003-204955 A (Toshiba Corp.),<br>22 July 2003 (22.07.2003),<br>paragraphs [0022] to [0065]<br>(Family: none) | 1-13 |
| A | JP 2009-201586 A (Fujifilm Corp.),<br>10 September 2009 (10.09.2009),<br>entire text; all drawings<br>(Family: none) | 1-13 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 August, 2011 (26.08.11) | 06 September, 2011 (06.09.11) |

| Name and mailing address of the ISA/<br>Japanese Patent Office | Authorized officer |
|---|---|
| Facsimile No. | Telephone No. |

Form PCT/ISA/210 (second sheet) (July 2009)

<table>
<tr><td colspan="2" align="center">**INTERNATIONAL SEARCH REPORT**</td><td>International application No.<br>PCT/JP2011/065153</td></tr>
</table>

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-203063 A (Siemens AG.),<br>16 August 2007 (16.08.2007),<br>entire text; all drawings<br>& JP 2007-203042 A     & JP 2007-203060 A<br>& JP 2009-525084 A     & US 2007/0183558 A1<br>& US 2007/0189449 A1   & US 2009/0154640 A1<br>& US 2010/0220834 A1   & EP 1803398 A1<br>& WO 2007/074029 A1    & WO 2007/087789 A1<br>& DE 102006017290 A    & CN 101011251 A<br>& AT 473685 T | 1-13 |
| A | JP 2009-11596 A (Konica Minolta Medical &<br>Graphic, Inc.),<br>22 January 2009 (22.01.2009),<br>entire text; all drawings<br>(Family: none) | 1-13 |

Form PCT/ISA/210 (continuation of second sheet) (July 2009)

## REFERENCES CITED IN THE DESCRIPTION

### Patent documents cited in the description

- WO 4445397 A
- JP 4445397 B
- JP 2009133823 A
- WO 2010050483 A

### Non-patent literature cited in the description

- **C. DAVID et al.** *Applied Physics Letters,* October 2002, vol. 81 (17), 3287
- **HECTOR CANABAL et al.** *Applied Optics,* September 1998, vol. 37 (26), 6227